(19) Europäisches Patentamt
European Patent Office
Office européen des brevets

(11) Publication number: 0 035 901
B1

(12) # EUROPEAN PATENT SPECIFICATION

(45) Date of publication of patent specification: **30.05.84**

(51) Int. Cl.³: **A 61 K 7/06**

(21) Application number: **81300971.9**

(22) Date of filing: **09.03.81**

(54) **Hair conditioning composition and method of conditioning hair therewith.**

(30) Priority: **10.03.80 US 128437**
**22.12.80 US 218372**
**13.02.81 US 234271**

(43) Date of publication of application:
**16.09.81 Bulletin 81/37**

(45) Publication of the grant of the patent:
**30.05.84 Bulletin 84/22**

(84) Designated Contracting States:
**AT BE CH DE FR GB IT LI LU NL SE**

(56) References cited:
**FR-A-2 162 464**
**FR-A-2 215 938**
**GB-A-2 025 228**

**Handbuch der Kosmetika und Riechstoffe, Bd III, 2. Aufl., S. 320-322; Bd. I, 3. Aufl., S. 73**

**The file contains technical information submitted after the application was filed and not included in this specification**

(73) Proprietor: **THE PROCTER & GAMBLE COMPANY**
**301 East Sixth Street**
**Cincinnati Ohio 45202 (US)**

(72) Inventor: **Bolich, Raymond Edward, Jr.**
**7201 Striker Road**
**Maineville Ohio 45039 (US)**

(74) Representative: **Brooks, Maxim Courtney et al**
**Procter & Gamble (NTC) Limited Whitley Road**
**Longbenton**
**Newcastle-upon-Tyne NE12 9TS (GB)**

**EP 0 035 901 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention is related to hair conditioning compositions which contain a volatile, liquid hair conditioning agent which is either a hydrocarbon or silicone and a hydrophobic thickener.

Volatile agents useful in hair conditioning compositions, while attractive for many reasons, present problems if it is desired to thicken them. Thickening is oftentimes attractive for cosmetic reasons.

The use of volatile agents in hair conditioning products is known. US—A 3,577,528 discloses two phase hair conditioners comprising an aqueous phase which contains a quaternary compound and a hydrocarbon or fluorinated hydrocarbon water immiscible phase. US—A 3,932,610 discloses a shampoo composition which may contain a volatile hydrocarbon solvent. US—A 3,818,105 discloses hair conditioners containing a $C_{12}$ to $C_{14}$ isoparaffinic hydrocarbon fraction. South African Patent Application 666421 discloses hair conditioners containing volatile silicones.

GB—A—2025228 discloses an aqueous conditioning and hairsetting composition containing a volatile silicone as conditioner, Carbopol® as viscosity controller and anionic and cationic resins as hairsetting components.

While these references disclose compositions which contain components of the type present in the compositions of the present invention, they are not entirely satisfactory, most often lacking in performance.

It is therefore an object of the present invention to provide hair conditioners which overcome problems associated with prior compositions.

It is a further object of the present invention to provide an improved method of conditioning hair.

These and other objectives will become more apparent from the disclosure which follows.

According to the present invention there is provided a hair conditioning composition for use as a rinse on freshly shampooed hair comprising from 1% to 99% of a liquid hair conditioning agent together with a thickening agent, the hair conditioning agent being selected from $C_{10}$—$C_{16}$ straight or branched chain hydrocarbons, cyclic polydimethylsiloxanes having from 3 to 7 silicon atoms and linear polydimethylsiloxanes having from 3 to 9 silicon atoms, the balance of the composition, if any, consisting of conventional components of hair conditioning compositions inclusive of water, ethanol, cationic hair conditioning agents, waxes, oils and perfumes, characterized by up to 1% of a hydrophobic thickening agent having a mass average molecular weight of from 50,000 to 5,000,000 selected from poly(1-butene), polyisoprene, polyisobutylene, polyvinyl isobutyl ether, poly(ethylene-ethyl acrylate) polyvinylethyl ether and polydimethylsiloxanes.

The compositions of the present invention comprise the above described essential components and may additionally contain several optional components. Each of the components is discussed in detail below.

Hair Conditioning Agent

The hydrocarbon and silicone agents useful in the present compositions have a boiling point in the range of from 99°C to 260°C and have a solubility in water of less than 0.1%. The hydrocarbons are either straight or branched chain and contain from 10 to 16, preferably from 12 to 16 carbon atoms. Examples of suitable hydrocarbons are decane, dodecane, tetradecane, tridecane and mixtures thereof.

The silicone useful in the composition of the present invention is either a cyclic or a linear polydimethylsiloxane. The number of silicon atoms in the cyclic silicones is from 3 to 7, more preferably 4 or 5.

The general formula for such silicones is

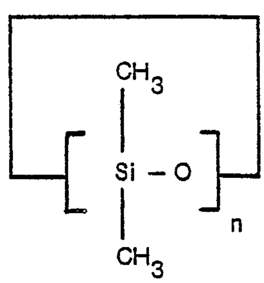

wherein n = 3—7

The linear polydimethylsiloxanes have from 3 to 9 silicon atoms and have the general formula

$$(CH_3)_3Si—O—[Si(CH_3)_2—O]_n—Si(CH_3)_3 \qquad n = 1—7$$

Silicones of the above type, both cyclic and linear, are offered by Dow Corning Corporation, Dow

Corning 344, 345 and 200 fluids, Union Carbide, Silicone 7202 and Silicone 7158, and Stauffer Chemical, SWS—03314.

The linear volatile silicones generally have viscosities of less than 5 centistokes at 25°C while the cyclic materials have viscosities less than 10 centistokes. "Volatile" means that the material has a measurable vapor pressure. A description of volatile silicones is found in Todd and Byers, "Volatile Silicone Fluids for Cosmetics", *Cosmetics and Toiletries,* Vol. 91, January, 1976, pp. 27—32, incorporated herein by reference.

The hair conditioning agent is present in the compositions of this invention at a level of from 1% to 99%, preferably from 2% to 60%, more preferably from 2% to 10%. The silicones are the preferred agents.

Hydrophobic Thickening Agent

The hair conditioning agents of the present invention are thickened with a hydrophobic thickening agent having a mass average molecular weight of from 50,000 to 5,000,000. The hydrophobic agents cause the hair conditioning agent to feel less oily on the hair.

Hydrocarbons are generally easier to thicken than the silicones although certain thickeners work equally well with either material. Examples of materials which work particularly well with hydrocarbons are poly(1-butene), polyisoprene, polybutadiene, ethylene/propylene copolymers, polyisobutylene, polyvinyl isobutyl ether and poly(ethylene-ethyl acrylate).

Thickeners which work particularly well with the silicones include the above mentioned polyvinyl isobutyl ether as well as polyvinyl ethyl ether and the polydimethylsiloxanes having a mass average molecular weight of up to about 5,000,000. The preferred thickeners are the polyvinylethers, most preferably polyvinyl isobutyl ether.

The hydrophobic thickening agent is present in the compositions of the present invention at a level of up to 1%, preferably from 0.005% to 1.0%, most preferably from 0.005% to 0.5%.

Optional Components

In addition to the above described essential components the compositions of the present invention may contain a wide variety of optional components. Some of the most preferred optional components are described in detail below.

Water is a preferred optional component in the present compositions. Water is particularly useful when certain of the other water soluble optional components described below are included. The amount of water is not critical but is generally at a level of up to 95%, preferably from 75% to 90%.

A water soluble thickening agent useful in the present compositions is a nonionic water soluble polymer. Included among such polymers are guar gum, locust bean gum, starches and starch derivatives such as hydroxyethyl amylose and starch amylose. Preferred polymers are guar gum and hydroxypropyl guar gum.

A cationic hair conditioning agent useful in the present compositions may be either a quaternary ammonium salt or the salt of a fatty amine.

Quaternary ammonium salts have the formula

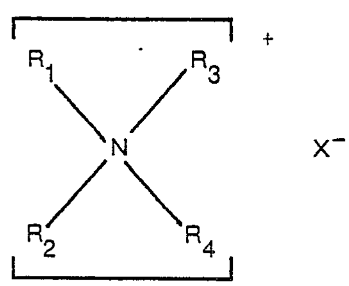

wherein $R_1$ is hydrogen, an aliphatic group of from 1 to 22 carbon atoms, or aromatic, aryl or alkaryl groups having from 12 to 22 carbon atoms, $R_2$ is an aliphatic group having 1—22 carbon atoms; $R_3$ and $R_4$ are each alkyl groups of from 1 to 3 carbon atoms, and X is a anion selected from halogen, acetate, phosphate, nitrate and methyl sulfate radicals. The aliphatic groups may contain, in addition to carbon and hydrogen atoms, ether linkages as well as amido groups among other groups.

Preferred quaternary ammonium salts are the dialkyl dimethyl ammonium chlorides, wherein the alkyl groups have from 12 to 22 carbon atoms and are derived from long-chain fatty acids, such as hydrogenated tallow. The term "tallow" refers to fatty alkyl groups derived from tallow fatty acids. Such fatty acids give rise to quaternary compounds wherein $R_1$ and $R_2$ have predominantly from 16 to 18 carbon atoms. The term "coconut" refers to fatty acid groups from coconut oil fatty acids. The coconut-alkyl $R_1$ and $R_2$ groups have from 8 to 18 carbon atoms and predominate in $C_{12}$ to $C_{14}$ alkyl groups.

Representative examples of quaternary ammonium salts of the invention include ditallow dimethyl ammonium chloride; ditallow dimethyl ammonium methyl sulfate; dihexadecyl dimethyl

ammonium chloride; di(hydrogenated tallow) dimethyl ammonium chloride; dioctadecyl dimethyl ammonium chloride; dieicosyl dimethyl ammonium chloride; didocosyl dimethyl ammonium chloride; di(hydrogenated tallow) dimethyl ammonium acetate; dihexadecyl diethyl ammonium chloride; dihexadecyl dimethyl ammonium acetate; ditallow dipropyl ammonium phosphate; ditallow dimethyl ammonium nitrate; di(coconutalkyl)dimethyl ammonium chloride; and stearyl dimethyl benzyl ammonium chloride.

Other quaternary ammonium salts useful herein are the compounds of the formula

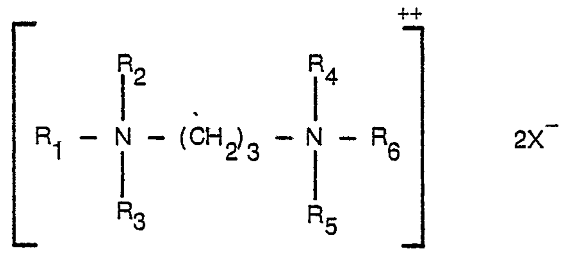

wherein $R_1$ is an aliphatic group having 16 to 22 carbon atoms, $R_2$, $R_3$, $R_4$, $R_5$ and $R_6$ are the same or different and are selected from H and alkyls having 1—4 carbon atoms and X is an anion as above defined. Tallow propanediammonium dichloride is an example of this quaternary ammonium salt.

Quaternary imidazolinium salts have the formula

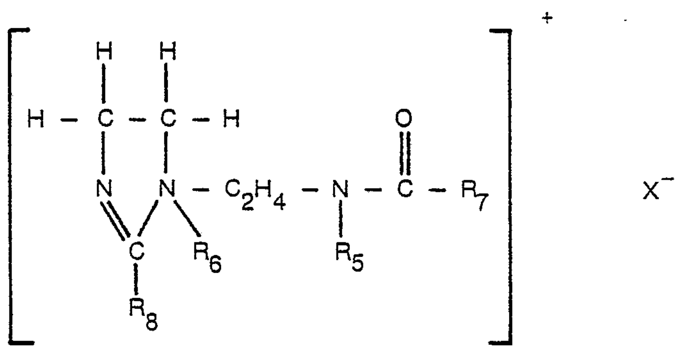

wherein $R_6$ is an alkyl group containing from 1 to 4, preferably from 1 to 2 carbon atoms, $R_5$ is an alkyl group containing from 1 to 4 carbon atoms or a hydrogen radical, $R_8$ is an alkyl group containing from 1 to 22, preferably at least 15 carbon atoms or a hydrogen radical, $R_7$ is an alkyl group containing from 8 to 22, preferably at least 15 carbon atoms, and X is an anion, preferably chloride. Other suitable anions include those disclosed with reference to the quaternary ammonium salts described hereinbefore.

Particularly preferred are those imidazolinium salts in which both $R_7$ and $R_8$ are alkyl of from 12 to 22 carbon atoms, e.g., 1 - methyl - 1 - [(stearoylamide)ethyl)] - 2 - heptadecyl - 4,5 - dihydroimidazolinium chloride; 1 - methyl - 1 - [(palmitoylamide)ethyl - 2 - octadecyl - 4,5 - dihydroimidazolinium chloride and 1 - methyl - 1 - [(tallowamide) - ethyl] - 2 - tallow - imidazolinium methyl sulfate.

Included as a suitable hair conditioner herein are salts of fatty amines. As used herein the amines may be primary, secondary or tertiary but the alkyl, substituted and unsubstituted groups preferably have from 12—22 carbon atoms. Preferred are the primary and secondary amines with the primary being the most preferred. Diamines having a long chain alkyl group may also be used. Examples of amines suitable for use include dimethyl stearamine, dimethyl soyamine, stearylamine, soyamine, myristylamine, tridecylamine, ethyl stearylamine, N-tallow propanediamine ethoxylated (5 moles E.O.) stearylamine dihydroxyethyl stearylamine and arachidylbehenylamine. The anions of the salts include those mentioned previously for the quaternary ammonium salts. Specific amine salts include stearylamine hydrochloride, soyamine chloride, stearylamine formate and N-tallow propanediamine dichloride.

The cationic hair conditioning agent, if present, is used at a level of from 0.05% to 4%, preferably from 0.1% to 2%.

Other optional components can be materials which are soluble in the volatile agent phase, in the aqueous phase or not soluble in either phase.

Included among materials soluble in the volatile agent phase are waxes such as cetyl alcohol and paraffin, and oils such as mineral oil and isopropyl myristate. Agents which are soluble in the aqueous phase include acrylamide and polyoxyethylene resins.

Among other optional components are dyes, perfumes, opacifiers, pearlescent aids, buffers, preservatives, antioxidants, and antidandruff aids such as zinc pyrithione and sulfur.

Method of Manufacture

There are many approaches suitable for making the present compositions. If it is desired to form an emulsion, the compositions should be processed in such a manner that the volatile agent is dispersed in the aqueous phase in particles of from 1 to 10 microns.

The preferred manner in which the hydrophobic thickener is incorporated into the present compositions is by preblending with the volatile liquid hair conditioning agent. This is accomplished by mixing the two together with agitation and heat until the thickener has completely dissolved. In the case of emulsions this preblend can be added to the aqueous phase or vice versa. Suitable proceses are shown in the Examples.

Industrial Applicability

The hair conditioning compositions of the present invention are preferably used as a rinse on freshly shampooed hair. The composition is used in an amount of from 1 g to 60 g, preferably from 2 g to 30 g and is then rinsed from the hair.

The following examples further describe and demonstrate embodiments within the scope of the present invention.

Unless otherwise indicated, all percentages herein are by weight.

Example I

The following composition is prepared:

| | | |
|---|---|---|
| Dow Corning Fluid 345[1] | | 7.00% |
| (cyclic silicone having 5 dimethyl siloxane groups) | | |
| Lutonal 1C 115[2] (polyvinyl isobutyl ether) | | 0.02 |
| Corn Starch Powder[3] | | 3.50 |
| Stearyl benzyl dimethyl ammonium chloride[4] | | 0.30 |
| Ethanol | | 7.00 |
| Distilled water | q.s. | 100.00% |

1 Supplied by Dow Corning Corporation.
2 Supplied by BASF.
3 Supplied by CPC International, Inc.
4 Supplied by Hexel-Fine Organics.

The above composition is prepared by dispersing 35 grams of starch powder in 822 grams of distilled water using a Lightnin® Mixer. The mixture is heated to about 65°C to fully dissolve and hydrate the starch. Three grams of the quaternary compound are then added, followed by the silicone/ether preblend. This preblend is prepared earlier by adding 0.2 g of Lutonal 1C 115 to 70 grams of Dow Corning Fluid 345 and heating this to 60°C with agitation for twelve hours by which time the ether is all dissolved. A high shear mixer, an Ultra Turrax® Model 45S4 dispersator supplied by Tekmer Company, was finally used to further reduce the silicone/ether particle size to 1 to 10 microns. The ethanol is added as the batch cools to room temperature.

Example II

The following composition was prepared:

| | | |
|---|---|---|
| Union Carbide 7158 Silicone Fluid[1] (cyclic silicone having 5 dimethyl siloxane groups) | | 8.000% |
| Lutonal 1C 115[1] (polyvinyl isobutyl ether) | | 0.008 |
| Jaguar HP—60[2] (hydroxypropyl guar gum) | | 1.100 |
| Ethanol | | 8.000 |
| Adogen 442[3] (90% active) | | 0.240 |
| Perfume | | 0.500 |
| Distilled Water | q.s. | 100.000% |

1 Supplied by Union Carbide Corporation.
2 Supplied by Stein-Hall.
3 Di hydrogenated tallow dimethyl ammonium chloride supplied by Sherex Chemical Company.

The above composition was prepared by placing 76.08 grams of the silicone, 400 grams of distilled water and 4 grams of a stock solution consisting of 2% Lutonal 1C 115 in the silicone into a mix tank. The stock solution had been prepared earlier by dissolving 2 grams of the ether in 98 grams of the silicone and mixing with a magnetic stirrer for 24 hours. A pre-mix was prepared by dispersing 2.4 grams of the quaternary and 11 grams of Jaguar HP—60 in 80 grams of ethanol. This was mixed with a magnetic stirrer for 10 minutes at ambient temperature. This pre-mix was then added to the main mix tank and sheared with a Ultra Turrax Model 45S4 for five minutes. To the batch were then added 421 grams of distilled water and 5 grams of perfume. A Lightnin® mixer was used to complete mixing the batch for 15 minutes.

**Claims**

1. A hair conditioning composition for use as a rinse on freshly shampooed hair comprising from 1% to 99% of a liquid hair conditioning agent together with a thickening agent, the hair conditioning agent being selected from $C_{10}$—$C_{16}$ straight or branched chain hydrocarbons, cyclic polydimethylsiloxanes having from 3 to 7 silicon atoms and linear polydimethylsiloxanes having from 3 to 9 silicon atoms, the balance of the composition, if any, consisting of conventional components of hair conditioning compositions inclusive of water, ethanol, cationic hair conditioning agents, waxes, oils and perfumes, characterized by up to 1% of a hydrophobic thickening agent having a mass average molecular weight of from 50,000 to 5,000,000 selected from poly(1-butene), polyisoprene, polyisobutylene, polyvinyl isobutyl ether, poly(ethylene-ethyl acrylate), polyvinylethyl ether and polydimethylsiloxanes.

2. A hair conditioning composition according to Claim 1 characterized in that the liquid hair conditioning agent is a hydrocarbon selected from the group consisting of decane, dodecane, tridecane, tetradecane and mixtures thereof.

3. A hair conditioning composition according to Claim 1 characterized in that the liquid hair conditioning agent is a silicone and is present at a level of from 2% to 60%.

4. A hair conditioning composition according to Claim 3 characterized in that the liquid hair conditioning agent is a cyclic silicone having either 4 or 5 dimethyl siloxane groups.

5. A hair conditioning composition according to any of Claims 1 to 4 characterized in that the amount of liquid hair conditioning agent is from 2% to 10% and the amount of hydrophobic thickening agent is from 0.005% to 0.5%.

6. A hair conditioning composition according to any of Claims 1 to 5 characterized in that the hydrophobic thickening agent is selected from polyvinylisobutyl ether, polyvinyl ethyl ether and mixtures thereof.

7. A hair conditioning composition according to any of Claims 1 to 6 characterized in that it additionally contains water an a cationic hair conditioning agent.

8. A method of conditioning hair characterized by:

I. applying from 1 g to 60 g of a composition according to any of Claims 1 to 7 to freshly shampooed hair and

II. rinsing the composition from the hair.

## Revendications

1. Composition de conditionnement des cheveux à utiliser comme produit de rinçage sur des cheveux fraîchement lavés, comprenant de 1% à 99% d'un agent liquide, de conditionnement des cheveaux, à côté d'un agent épaississant, l'agent de conditionnement des cheveux étant choisi parmi les hydrocarbures en $C_{10}$ à $C_{16}$ à chaîne droite ou ramifiée, les polydiméthylsiloxanes cycliques ayant de 3 à 7 atomes de silicium et les polydiméthylsiloxanes linéaires ayant de 3 à 9 atomes de silicium, le reste de la composition, s'il y en a, consistant en des composants traditionnels de compositions de conditionnement des cheveux, y compris l'eau, l'éthanol, des agents cationiques de conditionnement des cheveux, des cires, des huiles et des parfums, caractérisée en ce que l'agent épaississant est hydrophobe de masse moléculaire moyenne en masse de 50 000 à 5 000 000, choisi parmi le poly(1-butène), le polyisoprène, le polyisobutylène, le polyvinyl-isobutyléther, le poly(éthylène-acrylate d'éthyle), le polyvinyléthyléther et les polydiméthylsiloxanes et est en proportion jusqu'à 1%.

2. Composition de conditionnement des cheveux selon la revendication 1, caractérisée en ce que l'agent liquide de conditionnement des cheveux est un hydrocarbure choisi dans le groupe formé par le décane, le dodécane, le tridécane, le tétradécane et leurs mélanges.

3. Composition de conditionnement des cheveux selon la revendication 1, caractérisée en ce que l'agent liquide de conditionnement des cheveux est un silicone dont la teneur est comprise entre 2% et 60%.

4. Composition de conditionnement des cheveux selon la revendication 3, caractérisée en ce que l'agent liquide de conditionnement des cheveux est un silicone cyclique ayant 4 ou 5 groupes diméthylsiloxane.

5. Composition de conditionnement des cheveux selon l'une quelconque des revendications 1 à 4, caractérisée en ce que la proportion de l'agent liquide de conditionnement des cheveux est de 2 à 10%, et que la proportion de l'agent épaississant hydrophobe est de 0,005% à 0,5%.

6. Composition de conditionnement des cheveux selon l'une quelconque des revendications 1 à 5, caractérisée en ce que l'agent épaississant hydrophobe est choisi parmi le polyvinylisobutyléther, le polyvinyléthyléther et leurs mélanges.

7. Composition de conditionnement des cheveux selon l'une quelconque des revendications 1 à 6, caractérisée en ce qu'elle contient en outre de l'eau et un agent cationique de conditionnement des cheveux.

8. Procédé de conditionnement des cheveux, caractérisé en ce qu'il consiste:

I. à appliquer de 1 g à 60 g d'une composition selon l'une quelconque des revendications 1 à 7 à des cheveux fraîchement lavés, et

II. à éliminer par rinçage la composition des cheveux.

## Patentansprüche

1. Eine Zusammensetzung zum Konditionieren der Haare, für die Verwendung als eine Spülung an frisch shampooniertem Haar, enthaltend 1% bis 99% eines flüssigen Haarkonditioniermittels gemeinsam mit einem Verdickungsmittel, wobei das Haarkonditioniermittel aus gerad- oder verzweigtkettigen $C_{10}$—$C_{16}$-Kohlenwasserstoffen, cyclischen Polydimethylsiloxanen mit 3 bis 7 Siliciumatomen und linearen Polydimethylsiloxanen mit 3 bis 9 Siliciumatomen ausgewählt ist, während der gegebenenfalls vorhandene Rest der Zusammensetzung aus üblichen Komponenten von Zusammensetzungen zum Konditionieren der Haare, einschließlich Wasser, Ethanol, kationischen Haarkonditioniermittel, Wachse, Öle und Parfums, besteht, gekennzeichnet durch bis zu 1% eines hydrophoben Verdickungsmittels, das ein auf Masse bezogenes mittleres Molekulargewicht von 50.000 bis 5,000.000 aufweist und aus Poly(1-buten), Polyisopren, Polyisobutylen, Polyvinylisobutylether, Poly(ethylenethylacrylat), Polyvinylethylether und Polydimethylsiloxanen ausgewählt ist.

2. Eine Zusammensetzung zum Konditionieren der Haare nach Anspruch 1, dadurch gekennzeichnet, daß das flüssige Haarkonditioniermittel ein Kohlenwasserstoff ist, der aus der aus Decan, Dodecan, Tridecan, Tetradecan und Mischungen davon bestehenden Gruppe ausgewählt ist.

3. Eine Zusammensetzung zum Konditionieren der Haare nach Anspruch 1, dadurch gekennzeichnet, daß das flüssige Haarkonditioniermittel ein Silicon ist und in einer Menge von 2% bis 60% vorliegt.

4. Eine Zusammensetzung zum Konditionieren der Haare nach Anspruch 3, dadurch gekennzeichnet, daß das flüssige Haarkonditioniermittel ein cyclisches Silicon ist, das entweder 4 oder 5 Dimethylsiloxangruppen aufweist.

5. Eine Zusammensetzung zum Konditionieren der Haare nach irgendeinem der Ansprüche 1 bis 4, dadurch gekennzeichnet, daß die Menge an flüssigem Haarkonditioniermittel 2% bis 10% beträgt und die Menge an hydrophobem Verdickungsmittel 0,005% bis 0,5% ausmacht.

6. Eine Zusammensetzung zum Konditionieren der Haare nach irgendeinem der Ansprüche 1 bis 5, dadurch gekennzeichnet, daß das hydrophobe Verdickungsmittel aus Polyvinylisobutylether, Polyvinylethylether und Mischungen davon ausgewählt ist.

7. Eine Zusammensetzung zum Konditionieren der Haare nach irgendeinem der Ansprüche 1 bis 6, dadurch gekennzeichnet, daß sie zusätzlich Wasser und ein kationisches Haarkonditionniermittel enthält.

8. Ein Verfahren zum Konditionieren der Haare, gekennzeichnet durch:

I. das Aufbringen von 1 g bis 60 g einer Zusammensetzung nach irgendeinem der Ansprüche 1 bis 7 auf frisch shampoonierte Haare und

II. das Abspülen der Zusammensetzung aus den Haaren.